# FASCICULE DE BREVET EUROPEEN

(11) **EP 1 448 960 B1**
(45) Date de publication et mention de la délivrance du brevet: **29.03.2006**
(21) Numéro de dépôt: 02803833.9
(22) Date de dépôt: 27.11.2002
(51) Int. Cl.: G01G 19/44, G01G 19/50, A61B 5/053

(54) **APPAREIL DE MESURE D'UN PARAMETRE BIOLOGIQUE MUNI D'UN AFFICHEUR A REPRESENTATION GRAPHIQUE**
MIT EINER GRAPHISCHEN REPRÄSENTATIONSANZEIGE AUSGESTATTETE VORRICHTUNG ZUR MESSUNG EINES BIOLOGISCHEN PARAMETERS
APPARATUS FOR MEASURING A BIOLOGICAL PARAMETER EQUIPPED WITH A GRAPHIC REPRESENTATION DISPLAY

(30) Priorité: 30.11.2001 FR 0115493
(43) Date de publication de la demande: 25.08.2004
(73) Titulaire: SEB S.A., 69130 Ecully (FR)
(72) Inventeur: SIMOND, Bénédicte, F-74150 Bloye (FR); PATRAS, Yves, F-74330 Poisy (FR)
(74) Mandataire: Kiehl, Hubert
(86) Numéro de dépôt international: PCT/FR2002/004054
(87) Numéro de publication internationale: WO 2003/046493

(56) Documents cités:
- EP-A- 1 095 613
- EP-A- 1 120 083
- WO-A-00/58697
- WO-A-99/52425

## Description

La présente invention concerne un appareil de mesure d'un paramètre biologique, tels le poids, la composition corporelle, la taille, la température, la tension artérielle, le rythme cardiaque, etc, d'un individu, et elle concerne plus particulièrement un dispositif d'affichage à représentation graphique dudit paramètre.

Les appareils de mesure dits "numériques" des données biologiques d'un individu comportent, généralement, des moyens de mesure reliés à des moyens de mise en mémoire et/ou de calcul de la valeur dudit paramètre, ces derniers étant reliés à des moyens d'affichage de la valeur mesurée ou calculée sur la base des mesures effectuées par l'appareil. Les moyens d'affichage sont couramment constitués par un afficheur à cristaux liquides apte à indiquer la valeur mesurée dudit paramètre par une représentation alphanumérique ou par une représentation graphique, sous forme de juxtaposition de points, de courbe ou d'histogramme, voire par une combinaison des deux types de représentation.

Les valeurs mesurées d'un paramètre biologique sont différentes d'un individu à l'autre et elles varient dans le temps pour un même individu. Il est donc préférable qu'un tel appareil puisse comparer la valeur courante mesurée avec une autre valeur préenregistrée ou prédéterminée dans la mémoire de l'appareil et afficher ensuite une comparaison entre deux ou plusieurs valeurs ou une tendance d'évolution des valeurs du paramètre biologique mesuré.

On connaît, dans l'état de la technique, le document WO-A-0058697 qui est une balance de laboratoire prévue pour mesurer avec précision le poids d'un composant versé dans un récipient situé sur le plateau de mesure. Cet appareil comporte par ailleurs un afficheur à représentation graphique montrant le taux de variation du poids du composant au fur et à mesure que le récipient se remplit. L'afficheur est sous forme d'une zone rectangulaire au centre de la quelle se croisent deux axes orthogonaux représentant le poids cible. Un axe horizontal se déplace vers le centre de la zone d'affichage représentant un réglage grossier, alors qu'un axe vertical se déplace également vers le centre et représente un réglage plus fin de la quantité mesurée. Des limites de tolérance sont tracées à gauche et à droite du centre de la zone d'affichage, les valeurs de ces limites étant calculées en fonction de la vitesse de remplissage du récipient. Un tel afficheur est spécifique à une balance de laboratoire, son application à un appareil de mesure de paramètre biologique n'est ni prévue ni envisageable.

Le document US 4 113 039 décrit un appareil de mesure du poids du corps d'un individu, cet appareil étant muni d'un afficheur numérique du poids mesuré et de sa relation par rapport à un poids standard. Le poids standard est évalué par l'appareil pour chaque individu en fonction de sa taille, son age, son sexe et sa nationalité. Ensuite, l'afficheur indique sous forme numérique le poids mesuré et la différence par rapport au poids standard, une lampe témoin étant également actionnée pour signaler si le poids est normal, en dessous ou au dessus de la valeur standard calculée. Un tel afficheur numérique présente les inconvénients, d'une part, de faire appel à un écran d'assez grandes dimensions pour rendre lisibles les deux groupes de chiffres ce qui fait augmenter le gabarit de l'appareil et, d'autre part, de présenter un message uniquement numérique, donc peu compréhensible.

Dans le document WO 99/52425 est décrit un appareil de mesure de la composition corporelle d'une personne, cet appareil comportant un dispositif d'affichage combiné numérique et graphique des données mesurées et de leurs évolutions dans le temps. L'afficheur comporte une première zone d'affichage numérique des valeurs courantes du poids et de la masse grasse du corps de la personne et une deuxième zone de représentation graphique en utilisant des matrices de points, des deux paramètres et de leur évolution dans le temps. Un tel type d'afficheur fait appel à l'utilisation d'un grand nombre de points, ce qui rend l'afficheur complexe et coûteux.

Un appareil du même type comportant un afficheur combiné numérique et graphique a été exposé dans le document EP 1 120 083. La zone de représentation graphique est réalisée sous forme d'une matrice de segments rectangulaires allongés ayant le temps en abscisse et la valeur du paramètre mesuré en ordonnée. Cet afficheur permet d'indiquer la valeur courante d'un paramètre et de représenter son évolution dans le temps, la représentation pouvant se faire dans trois valeurs de graduation pour tenir compte des écarts dus à la fluctuation dans le temps des valeurs mesurées dudit paramètre. Cet afficheur montre l'évolution de la composition corporelle d'une personne de manière simplifiée par rapport au précédent, mais il s'avère cependant d'une utilisation contraignante, car il faut effectuer un nombre important de lectures afin de rendre l'interprétation compréhensible. De surcroît, avec un tel appareil, l'utilisateur peut mesurer la valeur courante d'un paramètre et la comparer avec les valeurs antérieures, sans pour autant savoir si la valeur mesurée ou son évolution se trouvent dans des limites de normalité adaptées à la personne qui utilise l'appareil.

Par ailleurs, le Dr. Boulier - Université Paris V - Paris - France est à l'origine d'un logiciel implémenté sur un support multimédia permettant d'afficher à l'écran d'un ordinateur, relié à un appareil de mesure d'un paramètre biologique, une représentation graphique simultanée des valeurs de plusieurs paramètres biologiques mesurés par l'appareil. La représentation des paramètres se fait par rapport à des limites théoriques, inférieure et supérieure, préétablies pour chaque paramètre. L'affichage de la valeur de chaque paramètre est réalisé sous forme d'un point sur une ligne comprenant un nombre pratiquement infini de points. Les limites inférieures et supérieures de tous les paramètres mesurés étant fixes, la représentation de chaque paramètre par rapport à ses limites respectives se fait par un ajustement de l'échelle de représentation de son axe. Ce logiciel permet déjà une représentation graphique simplifiée de plusieurs paramètres biologiques simultanément, mais il trouve ses limites lors de l'interprétation par l'utilisateur de l'image à l'écran. Ainsi, il s'est avéré que l'utilisateur a du mal à déchiffrer le message à l'écran, car il ne voit que des points sur des lignes, sans signification précise de la valeur des points qu'il regarde. De surcroît, une telle lecture nécessite beaucoup de concentration, ce qui, à terme, pourrait s'avérer fatigant pour l'utilisateur. En outre, ce logiciel nécessite un support multimédia performant distinct d'un pèse-personne ou d'un appareil simple de mesure de paramètre biologique.

Le but de la présente invention est de remédier aux inconvénients précités et de fournir un appareil de mesure d'un paramètre biologique d'un individu, comportant un afficheur permettant une représentation graphique précise tout en étant simplifiée, apte à faciliter l'interprétation par l'utilisateur des mesures effectuées.

Un autre but est un appareil de mesure d'un paramètre biologique d'un individu comportant un afficheur à représentation graphique des valeurs mesurées permettant leur comparaison avec des valeurs de normalité adaptées à l'individu.

Un autre but de l'invention est un appareil de mesure d'un paramètre biologique d'un individu, comportant un afficheur à représentation graphique de construction simplifiée et pouvant être fabriqué en grande série pour un coût réduit.

Ces buts sont atteints avec un appareil de mesure d'un paramètre biologique d'un individu comportant des moyens de mesure, des moyens de mise en mémoire et/ou de calcul des valeurs dudit paramètre reliés à un afficheur à représentation numérique et graphique du paramètre mesuré par rapport à un axe de représentation, du fait que ledit appareil comprend une unité de calcul qui évalue les valeurs de normalité dudit paramètre biologique de l'individu et que ledit afficheur représente le positionnement dudit paramètre mesuré par rapport à une zone de référence obtenue en délimitant par des repères fixes, situés de part et d'autre du centre dudit axe, un intervalle de normalité du paramètre mesuré en combinaison avec au moins une indication numérique adjacente.

Par zone de référence d'un paramètre biologique propre à l'individu on comprend une plage de valeurs démarquée par des valeurs évaluées par l'appareil ou choisies par l'individu en fonction de différentes données spécifiques à ce dernier. Ainsi, ces valeurs formant une zone de référence peuvent être des valeurs cibles choisies par l'individu pour améliorer ses performances ou, elles peuvent être représentées par des valeurs de normalité d'un paramètre. Ces valeurs de normalité peuvent être évaluées par une unité de calcul de l'appareil en fonction de différentes données conditionnelles, telles la taille, l'âge, le sexe, la forme physique, etc..

Par repère fixe d'un axe de représentation, on comprend un point situé sur un axe constitué d'une pluralité de points, repère qui est représenté toujours en une même position lors de la mise en route de l'afficheur. Par ailleurs, on peut également comprendre que ledit repère est appliqué ou gravé sur une partie supérieure de l'afficheur qui est superposée à celle contenant l'axe de représentation et laisse visible ledit axe, de manière à ce que ledit repère se confonde avec l'un des points dudit axe.

Selon l'invention, ledit afficheur est apte à ajuster le positionnement du paramètre mesuré par rapport aux deux repères fixes sur l'axe de représentation de manière à ce que la valeur mesurée puisse être figurée sur ledit axe par rapport à une zone de référence. Ainsi, la valeur mesurée peut être disposée de part ou d'autre de la zone de référence, c'est à dire à l'extérieur des repères fixes ou entre les deux repères, en fonction de la différence entre la valeur mesurée du paramètre et celle correspondant au repère le plus proche. Une telle représentation rend la signification de la valeur numérique affichée à côté plus compréhensible, tout en faisant appel à des moyens de représentation beaucoup plus simples, notamment un point sur un axe par rapport à une zone de référence.

Par conséquent, cette représentation graphique permet déjà de situer visuellement la position de chaque valeur par rapport à sa zone de référence et faciliter ainsi l'interprétation. De surcroît, en attribuant aux repères fixes des valeurs spécifiques, on arrive à représenter sur un même axe des valeurs d'un même paramètre pour plusieurs individus différents, car les repères fixes sont toujours représentés sur la même zone de référence, et seulement leurs valeurs affectées varient selon l'individu.

Par ailleurs, l'afficheur de l'invention comporte au moins une indication numérique adjacente à la zone de représentation graphique. Cette indication numérique est donc située dans une zone d'affichage numérique des valeurs mesurées et/ou calculées du paramètre biologique considéré, disposée dans la proximité de la zone d'affichage graphique afin de faciliter la lecture. Cette zone d'affichage numérique permet d'informer l'utilisateur de la valeur exacte du paramètre mesuré et représenté graphiquement et, par ailleurs, de vérifier la saisie correcte des données spécifiques à chaque utilisateur. Ainsi, la lecture des valeurs numériques combinée avec celle de la représentation graphique des mêmes valeurs par rapport à une zone de référence préétablie, permet de mieux situer visuellement le point sur le graphique, par rapport à ses limites de variation, tout en disposant de la signification numérique de ce point et/ou celle de ses limites. Ceci facilite l'interprétation par l'utilisateur des valeurs représentées à l'écran et permet d'améliorer la précision et la rapidité de la lecture.

L'appareil de l'invention comprend donc un afficheur simplifié, qui peut être intégré à l'appareil de mesure ou situé à distance par rapport à ce dernier, en étant relié à l'appareil par un câblage ou par un moyen de transmission à distance. Cet afficheur dispose d'une résolution simplifiée, par rapport à celle d'un écran d'ordinateur qui est quasiment infinie. L'afficheur de l'invention comporte un nombre optimum de segments calculé pour permettre, d'une part, une bonne interprétation des valeurs du paramètre mesuré et, d'autre part, un coût de fabrication réduit.

Utilement, lesdits moyens de calcul effectuent un calibrage de l'échelle de représentation graphique en fonction de la valeur attribuée à la largeur de la zone comprise entre les deux repères fixes.

L'ajustement du positionnement de la valeur mesurée se fait par un calibrage de l'échelle de représentation en considérant la valeur d'une graduation, ou valeur d'un point de l'axe, égale à la différence entre les valeurs attribuées à chacun des repères divisée par le nombre de graduations constituant, sur l'axe de représentation, la largeur de la zone délimitée par les deux repères fixes.

Avantageusement, lesdits repères fixes délimitent, de part et d'autre du centre dudit axe, un intervalle de normalité du paramètre mesuré.

Ainsi, les deux repères fixes forment les limites supérieure et inférieure de variation dudit paramètre dans une bande de normalité préétablie pour chaque individu. La bande de normalité peut être déterminée par des standards établis pour chaque individu en fonction de ses données personnelles (âge, sexe, taille, etc.) en prenant en considération la moyenne et les écarts-type calculés à partir de mesures dudit paramètre réalisées sur un échantillon statistiquement représentatif de chaque catégorie d'individus. Les deux repères fixes sont avantageusement positionnés de part et d'autre du centre de l'axe afin de faciliter l'interprétation et d'y pouvoir inscrire des variations dans les deux sens, croissant ou décroissant de variation du paramètre mesuré.

De préférence, ledit afficheur est apte à représenter graphiquement au moins deux paramètres distincts simultanément.

On peut ainsi représenter le positionnement de plusieurs paramètres d'ordre de grandeur différent par rapport à une même zone de normalité, ce qui permet une interprétation simultanée de la valeur de certains paramètres liés entre eux ou qui font référence à un même état de l'individu, par exemple le poids et la quantité de masse grasse.

Avantageusement, ledit afficheur est apte à afficher les indications numériques d'un seul paramètre à la fois, et il comprend des moyens pour basculer vers l'affichage des indications numériques d'un autre paramètre.

Ceci permet de simplifier l'afficheur qui comporte de ce fait un nombre moindre de segments. De surcroît, l'indication numérique peut occuper une zone plus importante de l'écran de l'afficheur, ce qui permet d'afficher l'indication numérique en grandes dimensions, mieux visibles par l'utilisateur.

De préférence, ledit afficheur est apte à afficher avec intermittence les indications numériques de chaque paramètre et il comporte un curseur reliant la représentation graphique à l'indication numérique dudit paramètre.

Ceci permet de faire défiler les valeurs numériques à l'écran, tout en regardant leur représentation graphique qui est permanente, afin de pouvoir comparer entre eux les deux paramètres mesurés. Un curseur est avantageusement situé adjacent à l'axe de représentation graphique de chaque paramètre, le curseur devenant actif au moment où l'affichage numérique indique les valeurs du paramètre représenté graphiquement afin de faire le lien entre la représentation graphique d'un paramètre donné et ses valeurs numériques affichées à l'écran.

Avantageusement, lesdits au moins deux paramètres sont représentés sur des axes parallèles.

Chaque paramètre est donc représenté sur un axe distinct, parallèle à l'axe de représentation de l'autre paramètre, ce qui facilite la lecture et la comparaison de deux paramètres l'un par rapport à l'autre.

De préférence, lesdits repères sont constitués par deux lignes parallèles qui croisent lesdits axes et définissent une zone de normalité pour les paramètres mesurés.

Ceci permet de constituer une même zone de normalité, qui a la même valeur pour tous les paramètres mesurés et qui permet en même temps l'analyse comparative des paramètres qui sont normalement exprimés dans des unités de mesure différentes.

Avantageusement, lesdites lignes parallèles sont verticales, ce qui implique une disposition des axes de représentations en position horizontale. Ceci permet de représenter une plage large de valeurs sur chaque axe, tout en utilisant un afficheur de dimensions réduites.

Dans un mode préféré de réalisation de l'invention, lesdits paramètres sont le poids, la quantité de masse grasse et la quantité de masse maigre de l'individu.

Avec un appareil selon l'invention on peut afficher graphiquement et simultanément plusieurs paramètres caractéristiques d'un état spécifique de l'individu, par exemple l'état de santé d'une personne est bien illustré par le poids, la quantité de masse grasse, le BMI représentant le rapport entre le poids et la taille au carré, la quantité d'eau dans les tissus, etc.. On préfère cependant comparer entre eux trois de ces paramètres, notamment le poids, la quantité de masse grasse et la quantité de masse maigre, car ce sont des paramètres connexes dont la comparaison permet à l'utilisateur d'établir son propre bilan de santé.

Dans un autre mode de réalisation de l'invention, lesdits paramètres sont la taille, le poids et l'indice de masse corporelle.

L'indice de masse corporelle, appelé aussi BMI, permet de déterminer, de manière simple et assez fiable, un excès ou une insuffisance de poids. L'indice de masse corporelle est calculé en rapportant le poids, en kilogrammes, au carré de la taille, en mètre. Ainsi, l'afficheur de l'invention permet une représentation simultanée de ces trois paramètres liés, représentatifs d'un état pondéral d'un individu, par rapport à une zone de normalité préétablie.

De préférence, l'axe de représentation graphique dudit afficheur est constitué d'une succession de 10 à 20 points.

Il a été constaté que sur un tel axe on peut représenter une gamme large de paramètres et leur zone de normalité, ce qui permet d'utiliser un afficheur comportant un nombre limité de points et de simplifier ainsi le circuit de commande de l'afficheur et d'en réduire le coût. Un tel afficheur peut être intégré à un appareil de mesure d'un paramètre biologique, sans pour autant augmenter de manière significative le coût de l'appareil.

De préférence, l'appareil selon l'invention est apte à représenter les paramètres biologiques spécifiques à plusieurs individus.

Ainsi, l'appareil est doté d'une mémoire de capacité suffisamment importante pour stocker les données faisant référence à plusieurs utilisateurs afin de reconnaître l'utilisateur ou d'enregistrer les mesures le concernant au fur et à mesure de leur collecte.

Avantageusement, l'appareil selon l'invention comporte des moyens de mise en mémoire des valeurs mesurées et d'affichage de l'historique de l'évolution sur une période prédéterminée.

On peut donc imaginer un afficheur comportant, pour chaque paramètre, une matrice de points avec, par exemple, un axe du temps dans l'abscisse et un axe des valeurs mesurées dans l'ordonnée. Ceci permet de visualiser l'évolution de chaque paramètre dans le temps et, en même temps, par rapport à la zone de normalité telle que définie précédemment.

Les caractéristiques de l'invention peuvent avantageusement être retrouvées dans un appareil de mesure de la composition corporelle d'un individu, comportant des moyens de mesure, des moyens de mise en mémoire et/ou de calcul des valeurs d'au moins un paramètre de la composition corporelle, reliés à un afficheur à représentation graphique du paramètre mesuré par rapport à un axe de représentation, du fait que ledit afficheur est apte à ajuster le positionnement dudit paramètre mesuré par rapport à une zone de référence délimitée de part et d'autre sur ledit axe par un repère fixe, en combinaison avec au moins une indication numérique adjacente.

Les caractéristiques de l'invention peuvent également être retrouvées dans un pèse-personne ou un pèse-enfant comportant des moyens de mesure d'un paramètre, tel le poids ou la taille, des moyens de mise en mémoire des valeurs mesurées et/ou de calcul, sur la base des valeurs mesurées et/ou saisies au moyen d'un clavier, des valeurs d'au moins un paramètre supplémentaire, ces moyens étant reliés à un afficheur à représentation graphique des paramètres mesurés et/ou calculés par rapport à un axe de représentation, caractérisé en ce que ledit afficheur est apte à ajuster le positionnement du paramètre mesuré et/ou calculé par rapport à une zone de référence délimitée de part et d'autre sur ledit axe par un repère fixe en combinaison avec au moins une indication numérique adjacente.

L'invention concerne également un procédé de mesure des paramètres biologiques d'un individu comportant des étapes de mesure des valeurs desdits paramètres, de mise en mémoire des valeurs mesurées et/ou de calcul des valeurs desdits paramètres, d'affichage graphique du paramètre mesuré par rapport à un axe de représentation, caractérisé en ce qu'il comporte une étape d'évaluation des valeurs de normalité dudit paramètre biologique et une étape de représentation du positionnement dudit paramètre mesuré par rapport à une zone de référence obtenue en délimitant, par des repères fixes situés de part et d'autre du centre dudit axe, un intervalle de normalité du paramètre mesuré en combinaison avec au moins une indication numérique adjacente.

D'autres caractéristiques et avantages de l'invention apparaîtront plus clairement à la lumière de la description et des dessins qui suivent illustrant, à titre d'exemple non limitatif, un mode de réalisation de l'invention. Ainsi, référence est faite aux figures 1 à 5, où :
- la figure 1 montre une vue de dessus d'un appareil selon l'invention;
- la figure 2 est un schéma bloc des principales parties constitutives de l'appareil;
- la figure 3 représente une vue de dessus d'un afficheur selon un premier mode de réalisation de l'invention;
- la figure 4 est une vue de dessus de l'afficheur de la figure 3 montrant un exemple d'affichage des valeurs mesurées ou calculées par l'appareil;
- la figure 5 est une vue de dessus d'un afficheur selon un deuxième mode de réalisation de l'invention, montrant un exemple d'affichage des valeurs mesurées ou calculées par l'appareil.

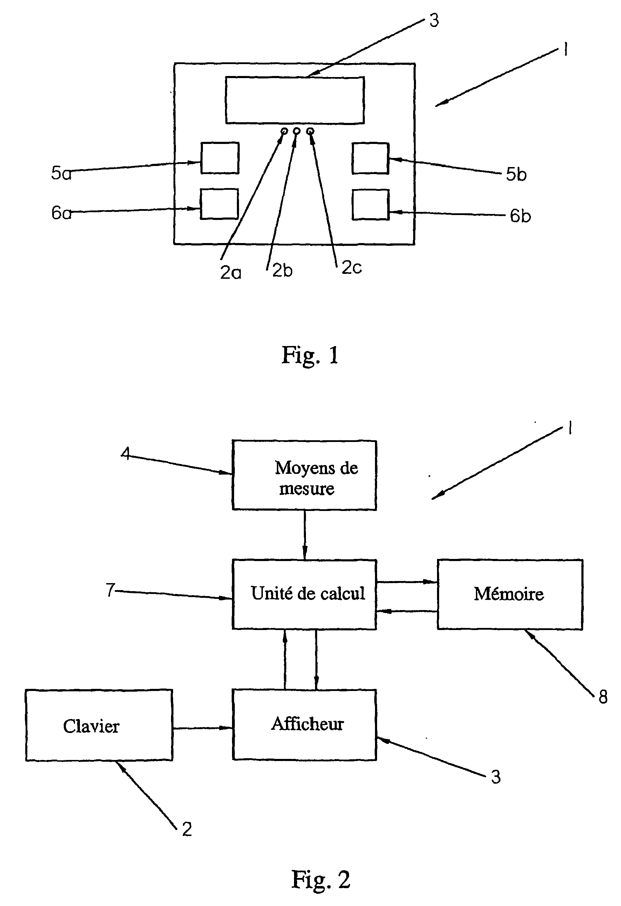

Dans les figures 1 à 4 est représenté un appareil de mesure 1 de la composition corporelle d'un individu basé sur la mesure d'une impédance bioélectrique. Un tel appareil utilise l'association d'un plateau de pèse-personne et d'une plaque munie de quatre électrodes. Deux de ces électrodes, dites d'excitation, 5a,5b sont associées à un circuit d'application d'un signal entre deux points du corps de l'individu, alors que les deux autres sont des électrodes de mesure 6a,6b. L'appareil de mesure 1 comporte des boutons de sélection 2a,2b,2c des valeurs spécifiques à l'individu qui sont ensuite affichées à l'écran de l'afficheur 3. Un tel type d'appareil est décrit plus en détail dans le document FR-A-2830740 au nom de la demanderesse.

A la figure 2 est représenté un schéma bloc de fonctionnement d'un tel appareil où les moyens de mesure 4 sont constitués par les cellules de pesée et les électrodes de l'appareil qui transmettent les signaux mesurés à une unité de calcul 7, notamment un microprocesseur ou microcontrôleur adaptés au traitement à effectuer. L'unité de calcul 7 est reliée à une mémoire 8 apte à stocker les données spécifiques à l'individu, telles les données mesurées par les moyens de mesure 4, ainsi que les données d'identification introduites par l'utilisateur au clavier 2. Les valeurs calculées par l'unité de calcul 7 ou stockées dans la mémoire 8 sont affichées par l'afficheur 3. Ces valeurs peuvent être le poids, la quantité de masse grasse, la quantité de masse maigre, le BMI (rapport entre le poids et le carré de la taille), le contenu d'eau de l'organisme, ou tout autre paramètre calculé sur la base de l'impédance bioélectrique du corps de l'individu.

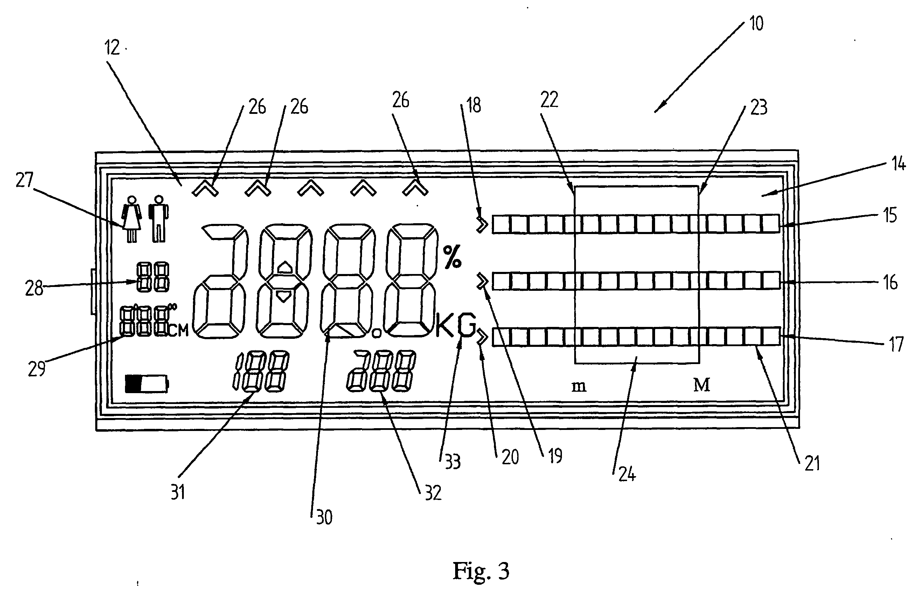

L'afficheur 3 comporte un écran d'affichage à cristaux liquides 10 qui est montré à la figure 3. L' écran d'affichage 10 de forme allongée comporte une première zone d'affichage numérique 12 et une deuxième zone d'affichage graphique 14 disposée dans le prolongement de la première.

Tel que visible à la figure 3, la zone d'affichage numérique 12 présente plusieurs groupes de chiffres ou de symboles constitués à base de segments qui montrent les valeurs spécifiques à l'utilisateur, saisies au clavier 2 ou mesurées par l'appareil 1. Ainsi, le champ 27 représente le sexe de l'utilisateur, le champ 28 indique l'âge et le champ 29 la taille en cm ou en pouces de l'utilisateur. Le champ 30 indique en grands caractères la valeur courante du paramètre mesuré ou calculé par l'appareil 1, valeur qui est suivie par l'unité de mesure associée au paramètre mesuré, représentée dans le champ 33. Les champs 31 et 32 montrent les valeurs des limites inférieure, respectivement supérieure de normalité du paramètre mesuré. Ces valeurs sont évaluées par l'unité de calcul 7 en fonction des données spécifiques à l'utilisateur, notamment la taille, l'âge, le sexe, préalablement saisies par l'utilisateur à l'aide du clavier 2 et rentrées dans la mémoire 8 de l'appareil 1. Dans la zone d'affichage numérique 12, des flèches 26 permettent à l'utilisateur de l'appareil de s'identifier, dans ce cas, les cinq curseurs témoignent de la mise en mémoire et de la sélection par la suite des données spécifiques à cinq utilisateurs.

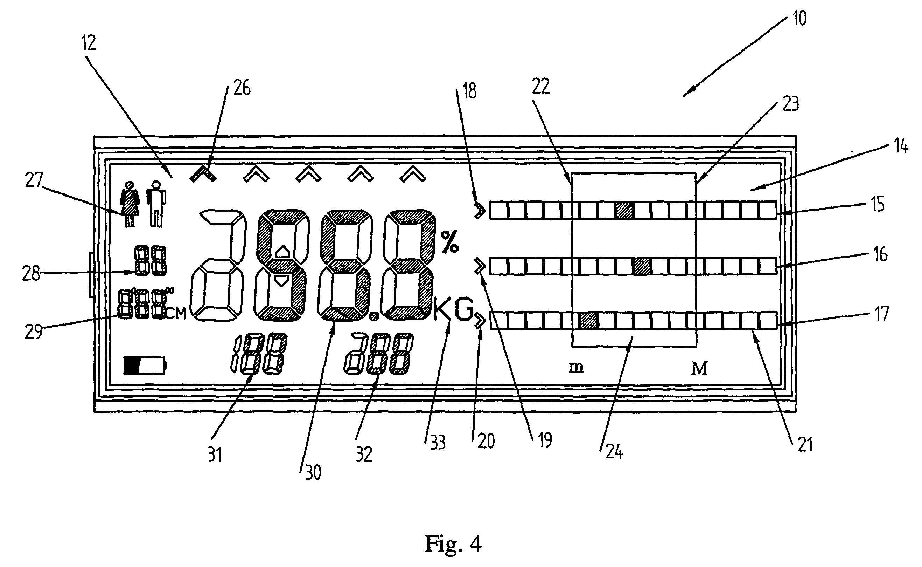

La zone d'affichage graphique 14 est constituée de trois axes parallèles horizontaux 15,16,17, chacun présentant un curseur 18,19,20 à l'une de ses extrémités. Les curseurs 18,19,20 font le lien avec la zone d'affichage numérique 12 en informant l'utilisateur sur le paramètre mesuré. Dans l'exemple présenté aux figures 3 et 4, sur l'axe 15 est représenté le poids de l'utilisateur, sur l'axe 16 la quantité de masse grasse et sur l'axe 17 la quantité de masse maigre.

Sur chacun des axes 15,16,17 est représentée la valeur courante d'un paramètre mesuré. Cette valeur est représentée par rapport à une zone de normalité 24 délimitée par deux lignes verticales 22,23 qui croisent les axes horizontaux 15,16,17. Chaque axe de représentation 15,16,17 est réalisé sous forme de ligne constituée d'une succession de segments ou points, comprise entre 10 à 20 points, de préférence 16 points. Les lignes verticales 22,23 délimitent sur chaque axe un intervalle de normalité d'une largeur comprise entre 5 et 7 points, ce qui permet de bien cadrer la valeur mesurée à l'intérieur ou de part et d'autre de l'intervalle de normalité pour tous les utilisateurs.

Les lignes verticales 22,23 sont de préférence marquées à demeure sur la partie de protection de l'écran à cristaux liquides 10. Cette partie de protection est de préférence transparente au moins partiellement pour permettre la lecture de l'écran 10. Les lignes verticales 22,23 peuvent être marquées à l'encre ou peinture, sérigraphiées ou gravées en relief par tout procédé mécanique ou chimique sur la partie supérieure de l'écran 10. Pour mieux marquer la zone de normalité 24, on préfère délimiter, sur la partie supérieure de l'écran 10, un rectangle ayant comme côtés les lignes 22,23. On peut également inscrire en la partie inférieure de chacune des lignes 22, 23, par le même procédé, les repères m et M correspondant à la limite inférieure, respectivement supérieure de la zone de normalité.

Lors de la première mise en marche de l'appareil, l'utilisateur commence par programmer ses données personnelles. Ainsi, il saisit ses données à l'aide du clavier 2, notamment le bouton 2b pour sélectionner les valeurs et les boutons 2a, 2c pour se déplacer dans le sens décroissant, respectivement croissant sur la partie numérique 12 de l'écran 10. Il saisit donc la taille dans le champ 29 l'âge dans le champ 28 et le sexe dans le champ 27, et il attribue ensuite une touche personnalisée à toutes ces données qui seront mémorisées et rappelées ensuite à chaque nouvelle utilisation de l'appareil par la même personne.

Un exemple d'utilisation de l'appareil est présenté à la figure 4, où la flèche 26 est allumée et correspond à l'utilisateur identifié par ses données inscrites aux champs 27,28 et 29. L'utilisateur est reconnu par l'appareil dès qu'il monte sur le plateau de pesée et mesure. Ses données personnelles s'affichent à l'écran et quelques instants après l'appareil mesure et calcule les données de la composition corporelle de l'individu. Ces données s'affichent dans la partie numérique 12 et dans la partie graphique 14.

A chaque prise de mesure, l'écran 10 de l'afficheur indique la valeur courante mesurée, en commençant par afficher le poids dans le champ 30, tel que visible à la figure 4. En même temps sont affichées les limites de normalité du poids de l'utilisateur, notamment la limite inférieure au champ 31 et la limite supérieure au champ 32. Ensuite, l'unité de calcul 7 effectue le calibrage de l'échelle de représentation graphique du poids sur l'axe 15. Pour ceci, à chaque point 21 de l'axe 15 lui est attribuée une valeur calculée en divisant la valeur de l'intervalle de normalité par le nombre de points correspondant à l'intervalle. Une fois l'échelle calibrée pour l'axe de représentation du poids 15, l'afficheur positionne la valeur courante mesurée sur l'axe 15 en allumant le point correspondant à ce positionnement. Le curseur 18 est également allumé pour prévenir l'utilisateur sur les valeurs numériques inscrites aux champs 30,31,32, sur leur correspondance avec les limites m, M et la représentation sur l'axe 15. L'utilisateur regarde ainsi les valeurs numériques dans la zone 12 et les compare à leur représentation graphique dans la zone 14.

Le même processus d'affichage est appliqué pour représenter les valeurs de la quantité de masse grasse sur l'axe 16, respectivement de la masse maigre sur l'axe 17.

L'affichage numérique des valeurs courantes et de leurs limites supérieure et inférieure peut avantageusement se faire en alternance, par une succession des valeurs de chaque paramètre dans un intervalle de temps prédéterminé. Pour avertir l'utilisateur sur le paramètre représenté dans la zone graphique 14, le curseur correspondant 18, 19 ou 20 s'allume et clignote, ainsi que le point de représentation sur l'axe de représentation 15,16 ou 17, alors que les valeurs numériques correspondantes sont inscrites successivement dans les champs correspondants de la zone numérique 12. On peut ainsi, par exemple, visualiser la représentation du poids courant de l'utilisateur clignotant sur l'axe 15 pendant 10 s, ensuite le point représentant la quantité de masse grasse clignotant sur l'axe 16 pendant une même période, et enfin le point de la quantité de masse maigre qui clignote pendant une même durée, alors que les deux autres restent allumés, mais fixes. Ainsi, les valeurs numériques et leurs représentations graphiques peuvent être visualisées et comparées en permanence pour chacun des trois paramètres mesurés.

Les points 21 restent allumés en permanence sur les axes 15,16,17, ce qui permet d'effectuer une comparaison des trois paramètres l'un par rapport à l'autre et par rapport à la zone de normalité qui a la même taille pour tous les paramètres. Ceci permet à chaque utilisateur d'établir son propre diagnostic et d'en suivre l'évolution, par exemple le glissement des valeurs par rapport aux limites de la zone de normalité. Ceci permet aussi de déterminer s'il y a déséquilibre ou pas entre les trois paramètres comparés. Ainsi, un alignement vertical dans la zone de normalité 24 des trois paramètres: poids, masse grasse et masse maigre peut témoigner d'une composition corporelle bien équilibrée.

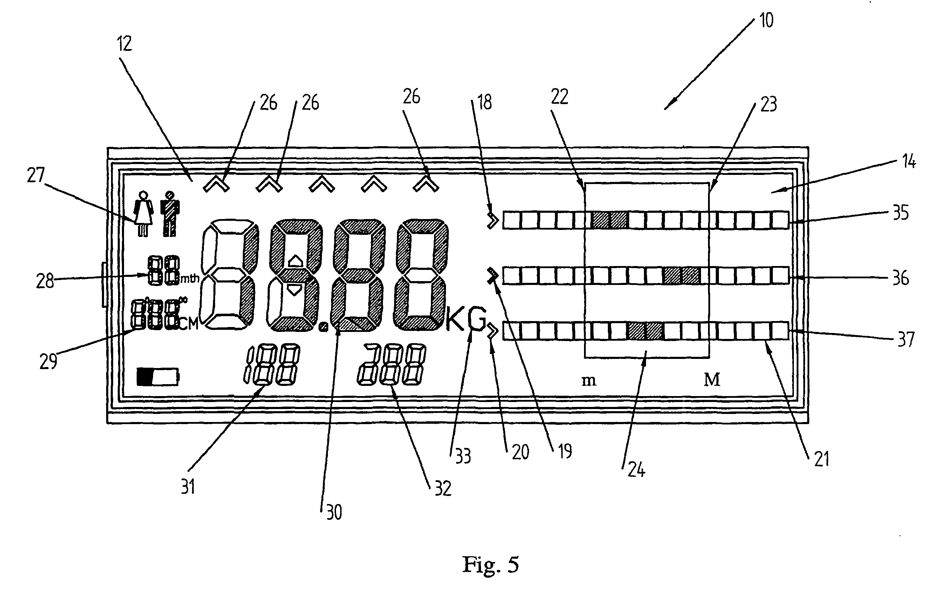

La figure 5 illustre un afficheur selon un deuxième mode de réalisation de l'invention, notamment un afficheur associé à un pèse-enfant ou à un pèse-bébé. Les moyens de mesure 4 associés à un tel appareil comprennent généralement un plateau de réception de poids supporté par des capteurs de poids à jauges de contrainte qui transmettent un signal électrique représentatif du poids mesuré à un circuit électronique comportant une unité de calcul 7, une mémoire 8 et un afficheur 3 relié à un clavier 2.

Un tel appareil de pesage peut intégrer, de manière avantageuse, une toise électronique ou un capteur de déplacement associé à un ensemble mobile se déplaçant par rapport à un repère fixe de l'appareil, permettant de mesurer automatiquement la taille de l'enfant qui se trouve sur le plateau de réception de poids et de la transmettre ensuite à l'unité de calcul 7 ou à la mémoire 8. Dans une variante, la taille connue est saisie directement au clavier 2 par le parent. Les autres caractéristiques de l'enfant (sexe, âge) sont également saisies au moyen du clavier 2 et mises en mémoire.

Tel que visible à la figure 5, l'écran d'affichage à cristaux liquide 10 comporte une zone d'affichage numérique 12 et une zone d'affichage graphique 14. La zone d'affichage numérique 12 permet d'afficher, d'une part, les valeurs saisies telles la nature du sexe de l'enfant 27, l'âge 28 et, d'autre part, les valeurs mesurées ou calculées par l'appareil, telles le poids, la taille ou l'indice de masse corporelle.

Le champ 30 indique, en caractères de grande taille, la valeur courante du paramètre mesuré ou calculé par l'appareil, avec son unité de mesure représentée en zone 33. Les champs 31 et 32 montrent les valeurs limites inférieure et supérieure statistiques ou de normalité du paramètre mesuré ou calculé, évaluées par l'unité de calcul 7, compte tenu de l'age, du sexe de l'enfant, voire de sa taille.

La zone d'affichage graphique 14 comporte trois axes parallèles horizontaux 35,36,37, chaque extrémité d'axe comportant, du côté de la zone d'affichage numérique 12 adjacente, un curseur 18,19,20 informant sur le paramètre qui a sa valeur affichée dans le champ 30. En figure 5, sur l'axe 35 est représentée la valeur de la taille mesurée par l'appareil ou saisie manuellement, sur l'axe 36 celle du poids mesuré par l'appareil et sur l'axe 37 celle de l'indice de masse corporelle calculé par l'appareil.

Les valeurs courantes mesurées, saisies ou calculées indiquées sur les axes 35,36,37 sont représentées par rapport à une zone de normalité 24 délimitée par un rectangle mis en évidence sur l'afficheur. Ainsi, comme dans le mode de réalisation précédemment décrit, l'échelle de représentation de chaque paramètre mesuré, saisi au clavier ou calculé par l'appareil est ajustée de façon à ce que les zones de normalité possèdent la même largeur. Ainsi, la ligne verticale 22 gauche de la zone de normalité 24 indique la limite inférieure théorique et la ligne verticale 23 droite de cette zone de normalité 24 indique la limite supérieure théorique.

Ainsi, l'afficheur de la figure 5 permet d'afficher simultanément de manière graphique, sur des axes parallèles, la taille de l'enfant, son poids et l'indice de masse corporelle calculé et de rapporter ces trois paramètres à une zone de normalité, tout en ayant la valeur numérique inscrite dans une zone d'affichage numérique adjacente. Ceci permet une meilleure visualisation des résultats et de voir comment l'enfant se place par rapport à une zone de normalité.

Un afficheur du type décrit en figure 5 n'est pas limité à l'utilisation dans le cadre d'un pèse-bébé ou d'un pèse-enfant, mais il peut être également appliqué à un pèse-personne, en adaptant la structure de l'appareil et les moyens de calcul pour afficher la taille, le poids et l'indice de masse corporelle d'un adulte.

D'autres variantes ou modes de réalisation de l'invention peuvent être imaginés sans sortir du cadre de ses revendications. Ainsi, on peut imaginer l'utilisation d'un afficheur selon l'invention avec un appareil d'une structure différente adapté à la mesure d'un paramètre biologique tels : la température, la tension artérielle, le pouls, le rythme cardiaque, etc. On pourrait également envisager de remplacer la représentation numérique par un affichage du type analogique commandé, par exemple, par un moteur pas-à-pas.

## Revendications

1. Appareil de mesure d'un paramètre biologique d'un individu comportant des moyens de mesure (4), des moyens de mise en mémoire (8) et/ou de calcul (7) des valeurs dudit paramètre reliés à un afficheur (3) à représentation numérique et graphique (14) du paramètre mesuré par rapport à un axe de représentation (15), **caractérisé en ce que** ledit appareil comprend une unité de calcul qui évalue les valeurs de normalité dudit paramètre biologique de l'individu et **en ce que** ledit afficheur (3) représente le positionnement dudit paramètre mesuré par rapport à une zone de référence obtenue en délimitant par des repères fixes (m, M), situés de part et d'autre du centre dudit axe (15), un intervalle de normalité (24) du paramètre mesuré en combinaison avec au moins une indication numérique adjacente (30,31,32).

2. Appareil selon la revendication 1, **caractérisé en ce que** lesdits moyens de calcul (7) effectuent un calibrage de l'échelle de représentation graphique en fonction de la valeur attribuée à la largeur de la zone comprise entre les deux repères fixes (m, M).

3. Appareil selon l'une des revendications précédentes, **caractérisé en ce que** ledit afficheur (3) représente graphiquement au moins deux paramètres distincts simultanément.

4. Appareil selon la revendication 3, **caractérisé en ce que** ledit afficheur (3) affiche les indications numériques d'un seul paramètre à la fois, et qu'il comprend des moyens pour basculer vers l'affichage des indications numériques d'un autre paramètre.

5. Appareil selon la revendication 4, **caractérisé en ce que** ledit afficheur (3) affiche avec intermittence les indications numériques de chaque paramètre et qu'il comporte un curseur reliant la représentation graphique à l'indication numérique dudit paramètre.

6. Appareil selon l'une des revendications 3 à 5, **caractérisé en ce que** lesdits au moins deux paramètres sont représentés sur des axes parallèles (15,16,17).

7. Appareil selon la revendication 6, **caractérisé en ce que** lesdits repères (m, M) sont constitués par deux lignes parallèles (22,23) qui croisent lesdits axes (15,16,17) et définissent une zone de normalité (24) pour les paramètres mesurés.

8. Appareil selon la revendication 7, **caractérisé en ce que** lesdites lignes parallèles (22,23) sont verticales.

9. Appareil selon l'une des revendications 3 à 8, **caractérisé en ce que** lesdits paramètres sont le poids, la quantité de masse grasse et la quantité de masse maigre de l'individu.

10. Appareil selon l'une des revendications 3 à 8, **caractérisé en ce que** lesdits paramètres sont la taille, le poids et l'indice de masse corporelle.

11. Appareil selon l'une des revendications précédentes, **caractérisé en ce que** l'axe de représentation graphique (15,16,17) dudit afficheur (3) est constitué d'une succession de 10 à 20 points.

12. Appareil selon l'une des revendications précédentes, **caractérisé en ce qu'**il est apte à représenter les paramètres biologiques spécifiques à plusieurs individus.

13. Appareil selon l'une des revendications précédentes, **caractérisé en ce qu'**il comporte des moyens de mise en mémoire des valeurs mesurées et d'affichage de l'historique de l'évolution sur une période prédéterminée.

14. Procédé de mesure des paramètres biologiques d'un individu comportant des étapes de mesure des valeurs desdits paramètres, de mise en mémoire des valeurs mesurées et/ou de calcul des valeurs desdits paramètres, d'affichage graphique du paramètre mesuré par rapport à un axe de représentation, **caractérisé en ce qu'**il comporte une étape d'évaluation des valeurs de normalité dudit paramètre biologique et une étape de représentation du positionnement dudit paramètre mesuré par rapport à une zone de référence obtenue en délimitant, par des repères fixes situés de part et d'autre du centre dudit axe, un intervalle de normalité du paramètre mesuré en combinaison avec au moins une indication numérique adjacente.

## Patentansprüche

1. Gerät zur Messung eines biologischen Parameters eines Individuums, mit Messmitteln (4), mit Mitteln zum Speichern (8) und/oder Berechnen (7) der Parameterwerte, die mit einer Anzeigeeinrichtung (3) mit digitaler und graphischer Darstellung (14) des gemessenen Parameters in Bezug auf eine Darstellungsachse (15) verbunden sind, **dadurch gekennzeichnet, dass** das Gerät eine Recheneinheit umfasst, die die Normalitätswerte des biologischen Parameters des Individuums berechnet, und dass die Anzeigeeinrichtung (3) die Position des gemessenen Parameters in Bezug auf einen Referenzbereich in Kombination mit mindestens einer benachbarten, digitalen Angabe (30, 31, 32) darstellt, wobei der Referenzbereich erhalten wird, indem ein Normalitätsintervall (24) des gemessenen Parameters durch feste Bezugspunkte (m, M) begrenzt wird, die sich auf der einen und auf der anderen Seite der Mitte der Achse (15) befinden.

2. Gerät nach Anspruch 1, **dadurch gekennzeichnet, dass** die Berechnungsmittel (7) eine Kalibrierung des graphischen Darstellungsmaßstabs in Abhängigkeit von dem Wert durchführt, der der Breite des Bereichs zwischen den beiden festen Bezugspunkten (m, M) zugeordnet ist.

3. Gerät nach einem der vorhergehenden Ansprüche, **dadurch gekennzeichnet, dass** die Anzeigeeinrichtung (3) mindestens zwei unterschiedliche Parameter gleichzeitig graphisch darstellt.

4. Gerät nach Anspruch 3, **dadurch gekennzeichnet, dass** die Anzeigeeinrichtung (3) die digitalen Angaben eines einzigen Parameters auf einmal anzeigt und Mittel für den Übergang zur Anzeige der digitalen Angaben eines anderen Parameters aufweist.

5. Gerät nach Anspruch 4, **dadurch gekennzeichnet, dass** die Anzeigeeinrichtung (3) die digitalen Angaben jedes Parameters intermittierend anzeigt und einen Cursor aufweist, der die graphische Darstellung mit der digitalen Angabe des Parameters verbindet.

6. Gerät nach einem der Ansprüche 3 bis 5, **dadurch gekennzeichnet, dass** die mindestens zwei Parameter auf parallelen Achsen (15, 16, 17) dargestellt sind.

7. Gerät nach Anspruch 6, **dadurch gekennzeichnet, dass** die Bezugspunkte (m, M) durch zwei parallele Linien (22, 23) gebildet sind, welche die Achsen (15, 16, 17) schneiden und einen Normalitätsbereich (24) für die gemessenen Parameter begrenzen.

8. Gerät nach Anspruch 7, **dadurch gekennzeichnet, dass** die parallelen Linien (22, 23) senkrecht sind.

9. Gerät nach einem der Ansprüche 3 bis 8, **dadurch gekennzeichnet, dass** die Parameter das Gewicht, der Fettanteil und der fettfreie Anteil des Individuums sind.

10. Gerät nach einem der Ansprüche 3 bis 8, **dadurch gekennzeichnet, dass** die Parameter die Größe, das Gewicht und der Körpermassenindex sind.

11. Gerät nach einem der vorhergehenden Ansprüche, **dadurch gekennzeichnet, dass** die Achse zur graphischen Darstellung (15, 16, 17) der Anzeigeeinrichtung (3) durch eine Folge von 10 bis 20 Punkten gebildet ist.

12. Gerät nach einem der vorhergehenden Ansprüche, **dadurch gekennzeichnet, dass** es dazu geeignet ist, die für mehrere Individuen charakteristischen biologischen Parameter darzustellen.

13. Gerät nach einem der vorhergehenden Ansprüche, **dadurch gekennzeichnet, dass** es Mittel zum Speichern der gemessenen Werte und Mittel zum Anzeigen des Entwicklungsverlaufs über einen vorbestimmten Zeitraum aufweist.

14. Verfahren zur Messung der biologischen Parameter eines Individuums, das die Schritte umfasst, in denen die Werte der Parameter gemessen werden, die gemessenen Werte gespeichert und/oder die Werte der Parameter berechnet werden, der gemessene Parameter in Bezug auf eine Darstellungsachse graphisch angezeigt wird, **dadurch gekennzeichnet, dass** es einen Schritt umfasst, in dem die Normalitätswerte des biologischen Parameters berechnet werden, und einen Schritt, in dem die Position des gemessenen Parameters in Bezug auf einen Referenzbereich in Kombination mit mindestens einer benachbarten, digitalen Angabe dargestellt wird, wobei der Referenzbereich erhalten wird, indem ein Normalitätsintervall des gemessenen Parameters durch feste Bezugspunkte begrenzt wird, die sich auf der einen und auf der anderen Seite der Mitte der Achse befinden.

## Claims

1. An instrument for measuring a biological parameter of a person, said instrument having measurement means (4), storage means (8) and/or computation means (7) for storing in a memory and/or for computing the values of said parameter, said storage means and/or computation means being connected to a display (3) for digitally representing the measured parameter and for graphically representing (14) said measured parameter relative to a representation axis (15), said instrument being **characterized in that** it has a computation unit that evaluates the normal values of said biological parameter of the person, and **in that** said display (3) represents the positioning of said measured parameter relative to a reference zone obtained by using unvarying markers (m, M) situated on either side of the center of said axis (15) to define a normal range (24) for the measured parameter, in combination with at least one adjacent digital indication (30, 31, 32).

2. An instrument according to claim 1, **characterized in that** said computation means (7) calibrate the scale of graphical representation as a function of the value attributed to the width of the zone lying between the two unvarying markers (m, M).

3. An instrument according to any preceding claim, **characterized in that** said display (3) graphically represents at least two distinct parameters simultaneously.

4. An instrument according to claim 3, **characterized in that** said display (3) displays the digital indications for one parameter at a time, and **in that** it has means for switching over to displaying the digital indications for another parameter.

5. An instrument according to claim 4, **characterized in that** said display (3) displays the digital indications for each parameter intermittently, and **in that** it has a cursor associating the graphical representation with the digital indication of said parameter.

6. An instrument according to any one of claims 3 to 5, **characterized in that** said at least two parameters are represented on parallel axes (15, 16, 17).

7. An instrument according to claim 6, **characterized in that** said markers (m, M) are constituted by two parallel lines (22, 23) that cross said axes (15, 16, 17) and define a normal zone (24) for the measured parameters.

8. An instrument according to claim 7, **characterized in that** said parallel lines (22, 23) are vertical.

9. An instrument according to any one of claims 3 to 8, **characterized in that** said parameters are the weight, the body fat mass, and the lean body mass of the person.

10. An instrument according to any one of claims 3 to 8, **characterized in that** said parameters are height, weight, and body mass index.

11. An instrument according to any preceding claim, **characterized in that** the graphical representation axis (15, 16, 17) of said display (3) is constituted by a succession of from 10 to 20 points.

12. An instrument according to any preceding claim, **characterized in that** it is suitable for representing the biological parameters specific to a plurality of people.

13. An instrument according to any preceding claim, **characterized in that** it has means for storing the measured values and for displaying the history of the variation in them over a predetermined period.

14. A method of measuring biological parameters of a person, said method comprising the steps of: measuring the values of said parameters; storing the measured values in a memory and/or computing the values of said parameters; and graphically displaying the measured value relative to a representation axis, said method being **characterized in that** it further comprises a step of evaluating the normal values for said biological parameter, and a step of representing the positioning of said measured , parameter relative to a reference zone obtained by using unvarying markers situated on either side of the center of said axis to define a normal range for the measured parameter, in combination with at least one adjacent digital indication.
